Europäisches Patentamt

European Patent Office

Office européen des brevets

(11) Publication number: **0 151 520**
**A1**

(19)

(12) **EUROPEAN PATENT APPLICATION**

(21) Application number: **85300330.9**

(22) Date of filing: **17.01.85**

(51) Int. Cl.⁴: **C 07 C 29/136**
C 07 C 29/14, C 07 C 35/08
C 07 C 31/12, C 07 C 33/18

(30) Priority: **18.01.84 GB 8401235**

(43) Date of publication of application:
**14.08.85 Bulletin 85/33**

(84) Designated Contracting States:
**BE DE FR GB IT NL**

(71) Applicant: **BP Chemicals Limited**
**Belgrave House 76 Buckingham Palace Road**
**London, SW1W 0SU(GB)**

(71) Applicant: **University of Sheffield**

**Sheffield S3 7HF(GB)**

(72) Inventor: **Maitlis, Peter Michael**
**University of Sheffield**
**Sheffield, S3 7HF(GB)**

(72) Inventor: **Smith, Thomas Ashley**
**55 Bakewell Road Hazel Grove**
**Stockport Cheshire, SK7 6JT(GB)**

(74) Representative: **Harry, John et al,**
**BP INTERNATIONAL LIMITED Patents Division Chertsey Road**
**Sunbury-on-Thames Middlesex TW16 7LN(GB)**

(54) Reactions involving methanol.

(57) A process for the hydrogenation of an aldehyde or a ketone comprises reacting the aldehyde or ketone with methanol in the presence of a platinum group metal catalyst. During the reaction methanol acts as a hydrogen donor and produces for example methyl formate as a coproduct along with the alcohol corresponding to the aldehyde or the ketone. The process can be operated either in the liquid phase using preferably a soluble catalyst or in the vapour phase using a supported platinum metal catalyst.

EP 0 151 520 A1

Croydon Printing Company Ltd

1

# REACTIONS INVOLVING METHANOL

The present invention relates to a process for the hydrogenation of an aldehyde or ketone. In particular the present invention relates to a process which uses methanol as a hydrogen donor molecule and which uses a platinum group metal catalyst.

The hydrogenation of olefinic double bonds in cycloolefins for example, cycloheptene to cycloheptane, using methanol as a hydrogen donor and $RhH(PPh_3)_4$ as catalyst, has been previously described in J Org Chem 1974 39, 1622.

The hydrogenation of one of the two olefinic double bonds in methyl linoleate has also been previously described using, various platinum phosphine complexes and stannous chloride as catalyst and employing methanol and benzene as hydrogen donor and solvent (J Am Chem Soc 1967 89 1592 to 1600).

Further, US Patent 4,290,961 discloses the reduction of carbonyl compounds using an alcohol as a hydrogen donor and employing, as catalyst, a rhodium or iridium complex which contains a chelating bidentate nitrogenous ligand together with, as promoter, a mineral alkali and/or a tertiary amine. In this reference, the alcohols described as hydogen donors include isopropanol, 2-butyl alcohol and ethanol. Methanol, however, is not described as a hydrogen donor and, so far as it is referred to at all, is described as an inert solvent.

It has now been found that saturated or unsaturated aldehydes and ketones can be hydrogenated using methanol as a hydrogen donor and, as a catalyst, a platinum group metal.

Accordingly, the present invention provides a process for the hydrogenation of an aldehyde or a ketone, which process comprises reacting the aldehyde or ketone with methanol, in the presence of an effective amount of a platinum group metal catalyst, under conditions which cause transfer of hydrogen from the methanol to the aldehyde or the ketone.

The aldehyde or ketone may in principle be any aldehyde or ketone. Simple aliphatic aldehydes and ketones can be used for example those aldehydes and ketones having up to 20 carbon atoms. Preferred aldehydes and ketones are those having up to 10 carbon atoms. In addition to aliphatic aldehydes and ketones, aromatic aldehydes and ketones e.g. benzaldehyde, acetophenone, and the like can be used. In the case of ketones, the keto group may form part of a cyclic compound such a cyclohexanone, cycloheptanone, or a substituted derivative thereof acetaldehyde, acetone, MEK or MiBK.

The aldehyde or ketone may also contain other functional group and may also have additional unsaturation. Thus methyl vinyl ketone can be hydrogenated using the process described above. In such cases both the aldehyde or keto group and the additional unsaturation can be hydrogenated.

The products of the process described above comprises the alcohol derivable from the aldehyde or ketone by hydrogenation and a dehydrogenation product of methanol, for example methyl formate. Thus if the ketone used is acetone the alcohol product is isopropanol.

The reaction described above is catalysed by a platinum group metal catalyst. By a platinum group metal catalyst is meant a catalyst having one or more of the metals ruthenium, rhodium, palladium, osmium, iridium and platinum present. The preferable platinum group metals are ruthenium and rhodium.

The platinum group metal catalyst can be a simple salt or an inorganic or organometallic complex of the platinum group metal. Such complexes may contain ligands such as a halide ions, amines, phosphines, phosphites, arsines, and stibines. In addition ligands able to Π-bond to the metal, e.g. the cyclopentadienyl and pentamethyl cyclopentadienyl anions and benzene and its substituted derivatives can also be present. The complex can be cationic, anionic or neutral and can be prepared separately or generated in situ in the reaction mixture. For example the complex can be generated in situ by reacting a phosphine or phosphite with a simple salt of ruthenium or rhodium.

The phosphine or phosphite can be a monodentate, bidentate or polydentate phosphine or phosphite. Preferred phosphines include trialkyl phosphines, e.g. tributylphosphine, trialylphosphines, e.g. triphenylphosphine, and phosphines containing both alkyl and aryl groups attached to the phosphorus atom e.g. diphos, dppm and the like. Preferred phosphites include trialkylphosphite and triarylphosphites.

When salts and complexes of the type described above are used it is preferable to operate the process as a liquid phase process with the catalyst souble or partially soluble in the reaction mixture.

As an alternative to a liquid phase process, the hydrogenation may be effected in the gas phase. In this mode of operation the aldehyde and ketone, together with the methanol, are vapourised and passed over or through a bed of solid catalyst. Suitably the solid catalyst comprises the plantinum group metal supported on an inert carrier. The carrier can be inorganic, for example, a metal oxide, silica, alumina, an aluminosilicate, clay or diatomaceous earth or organic for example polymers, such as polystyrene, polystyrene/divinylbenzene copolymers, fluorinated polymers.

Various methods of supporting the platinum group metal, on the inert carrier are available and are familiar to the skilled man. Examples include impregnation, ion-exchange and coprecipitation methods. A preferred method involves chemically binding a ligand to the inert carrier, for example a phosphine ligand, and then complexing the bond phosphine ligand with the platinum group method. The phosphine is therefore bound to the carrier by means of one or more reactive groups attached to the phosphine.

However, for both modes of operation it is preferable that than the molar ratio of methanol to aldehyde or ketone is greater than 1:1 preferably 10:1.

The reaction is suitably carried out at elevated temperature in the range 100–240°C although higher temperatures may be used if desired. Preferaby the reaction temperature should lie in the range 140–185°C.

The reaction may be carried out at pressures up to 20 bars, preferably in the range 5–10 bars. When the reaction is carried out at superatmospheric pressure, the pressure may be generated by the autogenous pressure of one or more of the reactants at the temperature of reaction, or it may be generated by applying an overpressure of a suitable inert gas e.g. nitrogen, or a combination of both.

The process as described can be carried out either batchwise or continuously. When the process is operated in the gas phase, continuous operation is preferred.

The invention is illustrated by the following examples.

Examples 1 to 9

Cyclohexanone (8 mmol) and methanol (195 mmol) and catalyst (0.08 mmol) were heated to 150°C in a Fisher-Porter tube. At the end of the reaction time recorded in Table 1 below, the contents were cooled and analysed by gas chromatography.

In Examples 6–8 the ruthenium trichloride used was ruthenium chloride hydrate supplied by Johnson-Matthey. The catalysts therefore refer to one mole of ruthenium chloride hydrate and the appropriate number of moles of triphenylphosphine.

Examples 1 to 9 illustrate the following reaction using different catalysts and varying reaction times.

Cyclohexanone + methanol $\longrightarrow$ cyclohexanol + methyl formate.

TABLE 1

| Example | Catalyst | Reaction time (hours) | Cyclohexanol mmol and moles of product per mole of catalyst | Methyl formate mmol and moles of product per mole of catalyst |
|---|---|---|---|---|
| 1 | $[(C_5Me_5Rh)_2Cl_4]$ | 19 | 1.1 (14) | 1.3 (16) |
| 2 | $[(C_5Me_5Ir)_2Cl_4]$ | 18 | 1.2 (15) | trace. |
| 3 | $[(C_6Me_6Ru)_2Cl_4]$ | 18 | 0.5 (6) | trace. |
| 4 | $(Ph_3P)_3RuCl_2$ | 9.5 | 6.8 (85) | 4.8 (60) |
| 5 | $(Ph_3P)_3OsHBr(CO)$ | 18 | 1.0 (12) | 2.8 (35) |
| 6 | $RuCl_3+1molePPh_3$ | 18 | 0.3 (3) | 3.3 (42) |
| 7 | $RuCl_3+2molesPPh_3$ | 18 | 0.3 (4) | 3.6 (45) |
| 8 | $RuCl_3+3molesPPh_3$ | 18 | 6.2 (77) | 3.5 (44) |
| 9 | $(p-cymene)_2Os_2Cl_4$ + 4 moles $PPh_3$ | 18 | 1.7 (21) | 0.6 ( 7) |

TABLE 2

| Example | Hydrogen Acceptor | Product | mmol of product and moles/mole catalyst | Methyl formate mmol and moles/mole catalyst |
|---|---|---|---|---|
| 10 | cyclohexanone | cyclohexanol | 5.4 (67) | 2.0 (25) |
| 11 | PhCOMe | PhCHOHMe | 0.4 (5) | 2.1 (26) |
| 12 | MeCOEt | MeCHOHEt | 1.5 (19) | 1.5 (18) |
| 13 | PhCHO | PhCH$_2$OH | 1.4 (17) | trace. |
| 14 | 4t-butyl cyclohexanone | 4-t butyl cyclohexanol (trans:cis=4:1) | 6.2 (77) | 0.7 (9) |

## Examples 10 to 14

These examples illustrate the reaction of methanol as a hydrogen donor with various hydrogen acceptors using, as catalyst, $(Ph_3P)_3RuCl_2$ which was the most active catalyst recorded in Table 1. The reaction may be summarised:

methanol + hydrogen acceptor $\longrightarrow$ product + methyl formate.

In the examples the hydrogen acceptor (8 mmol), methanol (200 mmol) and $(Ph_3P)_3RuCl_2$ (0.08 mmol) were heated in a Fisher-Porter tube at 139 to 148°C for 18 hours. At the end of the reaction time the contents were cooled and analysed by gas chromatography.

Example 15

· Cyclohexanone (4.9 cm$^3$, 48 mmol) methanol (55 cm$^3$) and [(C$_5$Me$_5$Rh)$_2$(OH)$_3$]Cl (0.3 g, 0.5 mmol) were heated in a stainless steel autoclave under nitrogen for 5 hours at 150°C and 60 bar. Analysis showed the presence of cyclohexanol (4.1 mmol; 8 mol/mol catalyst) and methyl formate (2.1 mmol; 4 mol/mol catalyst).

Example 16

Methylvinylketone (3.9 cm$^3$, 48 mmol) methanol (56 cm$^3$) and [(C$_5$Me$_5$Rh)$_2$(OH)$_3$]Cl (0.3 g, 0.5 mmol) were heated in a stainless steel autoclave under nitrogen for 5 hours at 150°C and 60 bar. Analysis showed the presence of methylethylketone (butan-2-one) (25 mmol; 52 mol/mol) catalyst and methyl formate (2.1 mmol; 4 mol/mol catalyst).

Example 17

Cyclohexanone (8 mmol), methanol (195 mmol), ruthenium trichloride (hydrate) (0.08 mmol) and trimethylphosphite (0.24 mmol) were heated in a glass liner in a rocking autoclave (135°/18 h). Analysis showed the presence of cyclohexanol (7.0 mmol; 87 mol/mol catalyst).

Example 18

The method of Example 17 was used except that a bidentate phosphine was used in place of trimethylphosphite. Cyclohexanol (7.2 mmol; 92 mmol/mol catalyst) was formed.

Example 19

Cyclohexanone (8 mmol), methanol (195 mmol) and [Ru(PPh$_3$)$_2$Cl$_3$(NO)] (0.08 mmol) were heated in a glass liner in a rocking autoclave (150°C/18 h). Cyclohexanol (7.4 mmol; 94 mol/mol catalyst) was formed.

Example 20

Example 20 illustrates that the process of the present invention may be carried out in the gas phase.

(a) Preparation of the Catalyst

Davison 57 grade silica gel (mesh size 30-60) was functionalised by reaction with the phosphine (EtO)$_3$CH$_2$CH$_2$PPh$_2$. The functionalised silica (10 g, 0.8% wt P) was treated with RuCl$_3$ in 200 mls of refluxing ethanol. After ca 2 hrs the mixture was cooled, the solid separated by filtration and the remaining solvent removed under vacuum. Care was taken to exclude air at all times during the preparation. The resulting catalyst was bottle-green.

(b) Gas phase Process

4 g of the catalyst described in (a) was charged to a tubular glass reactor (0.5 cm Ld) contained in an oven and equipped with preheat section, liquid feed pump, gas feed system and product recovery section. Nitrogen (30 ml/min) and a 1:1 mixture of methanol and acetone (liquid feed equivalent to 15 ml gas/min) were then passed over the catalyst. During this time the catalyst was maintained at 180°C. After ca 4 hrs the feed was stopped and the recovered product analysed. The product contained 0.5% wt methyl formate and 0.9 wt% isopropanol as determined by GC.

Claims:

1. A process for the hydrogenation of an aldehyde or a ketone which process comprises reacting the aldehyde or the ketone with methanol in the presence of an effective amount of a platinum group metal catalyst, under conditions which cause transfer of hydrogen from the methanol to the aldehyde or the ketone.

2. A process as claimed in Claim 1 characterised in that the reaction is carried out in the liquid phase using a platinum group metal catalyst which is soluble in the reaction mixture.

3. A process as claimed in Claim 2 characterised in that the platinum group metal catalyst is a simple salt, or an inorganic or organometallic complex of a metal selected from the group consisting of ruthenium, rhodium, palladium, osmium, iridium, and platinum.

4. A process as claimed in Claim 3 characterised in that the platinum group metal catalyst is a phosphine or phosphite complex of ruthenium or rhodium.

5. A process as claimed in Claim 4 characterised in that the phosphine or phosphite complex of ruthenium or rhodium has at least one phosphine or phosphite ligand selected from the group consisting of trialkylphosphines, triarylphonsphines, trialkylphosphites and triarylphosphites.

6. A process as claimed in Claim 3 characterised in that the phosphine or phosphite complex is formed <u>in situ</u> from a ruthenium or osmium compound and a phosphine.

7. A process as claimed in Claim 1 characterised in that the reactants are vapourised and passed over or through a bed of the platinum group metal catalyst.

8. A process as claimed in Claim 7 characterised in that the platinum group metal catalyst comprises a platinum group metal support on an inert carrier.

9. A process as claimed in Claim 8 characterised in that (i) the inert carrier is functionalised by chemically bonding a ligand to the inert carrier and (ii) the platinum group metal is subsequently complexed to the ligand.

10. A process as claimed in Claim 7 characterised in that the platinum group metal catalyst is a ruthenium catalyst.

**European Patent Office**

**EUROPEAN SEARCH REPORT**

0151520
Application number

EP 85 30 0330

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| A | US-A-2 156 217 (CH.E. ANDREWS) * Page 1, left-hand column, lines 1-46; page 2, table * | 1 | C 07 C 29/136 <br> C 07 C 29/14 <br> C 07 C 35/08 <br> C 07 C 31/12 <br> C 07 C 33/18 |
| A | CHEMISTRY LETTERS, no. 3, March 1982, pages 261-264, The Chemical Society of Japan, Tokyo, JP; K. TANI et al.: "Rh(I) complexes containing fully alkylated mono- and diphosphine ligands as highly active hydrogenation catalysts for carbonyl compounds" | 1 | |
| A | BULLETIN OF THE ACADEMY OF SCIENCE OF THE USSR, vol. 26, no. 4, point 1, April 1977, pages 666-670; V.Z. SHARF et al.: "Mechanism of transfer of hydrogen from an alcohol to a ketone in the presence of complexes RhCl(PPh)3 and RuCl2(PPh3)3" | 1 | TECHNICAL FIELDS SEARCHED (Int. Cl.4) <br><br> C 07 C 29/00 <br> C 07 C 31/00 <br> C 07 C 67/00 <br> C 07 C 69/00 |

The present search report has been drawn up for all claims

| Place of search | Date of completion of the search | Examiner |
|---|---|---|
| THE HAGUE | 29-04-1985 | KINZINGER J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82